# EUROPÄISCHE PATENTSCHRIFT

(11) **EP 3 691 722 B1**
(45) Veröffentlichungstag und Bekanntmachung des Hinweises auf die Patenterteilung: **18.01.2023**
(21) Anmeldenummer: 18785567.1
(22) Anmeldetag: 05.10.2018
(51) Int. Cl.: A61M 15/00, B05B 1/34, B05B 15/40, A61M 11/00, B65D 83/14, B65D 83/64

(54) **DÜSENKÖRPER UND SPRÜHVORRICHTUNG**
NOZZLE BODY AND SPRAYING DEVICE
CORPS D'AJUTAGE ET DISPOSITIF DE PULVÉRISATION

(30) Priorität: 06.10.2017 DE 202017005165 U
(43) Veröffentlichungstag der Anmeldung: 12.08.2020
(73) Patentinhaber: Werrta GmbH Düsen- und Zerstäubungstechnik, 14089 Berlin (DE)
(72) Erfinder: RENTSCH, Rüdiger, 14089 Berlin (DE)
(74) Vertreter: Kehl, Ascherl, Liebhoff & Ettmayr Patentanwälte Partnerschaft mbB
(86) Internationale Anmeldenummer: PCT/EP2018/077158
(87) Internationale Veröffentlichungsnummer: WO 2019/068878

(56) Entgegenhaltungen:
- EP-A1- 3 097 981
- DE-A1-102006 010 877
- DE-U1-202017 002 851
- US-A- 5 497 944

## Beschreibung

Die vorliegende Erfindung betrifft einen Düsenkörper, insbesondere auch für den Sprühkopf einer Sprühvorrichtung, beispielsweise einer Sprühdose.

Düsenkörper werden in der Technik häufig eingesetzt, wenn Fluide wie Gase, Flüssigkeiten und Aerosole von einer Zuleitung oder einem Vorlagebehälter aus zerstäubt, d.h. in ein Aerosol überführt, und dosiert, d.h. mit einem innerhalb eines vorgegebenen Bereich liegenden Durchsatz, ausgetragen werden sollen. Das jeweilige Fluid oder Fluidgemisch wird dem Düsenkörper dabei durch eine oder mehrere Eintrittsöffnungen aus der entsprechenden Zuleitung oder Vorlage aus zugeführt und verlässt ihn durch einen oder mehrere Austrittskanäle. Vielfältige Anwendungen sind dem Fachmann bekannt aus Sprühsystemen wie beispielsweise Inhalatoren oder einfachen Sprühdosen, Einspritzsystemen etc.

Hinreichend kleine Tröpfchen, wie sie insbesondere zum Erzielen von Lungengängigkeit im medizinischen Bereich erforderlich sein können, lassen sich nur erzeugen, wenn auch die Strukturen im Sprühkopf hinreichend klein sind. Auch die Reduzierung des Fluiddurchsatzes bei vorgegebenem Druckgefälle erfordert entsprechend kleine Strukturen im Sprühkopf.

Neben der erzielbaren Tröpfchengröße und dem sich einstellenden Durchsatz ist auch die Breite der Tröpfchengrößenverteilung ein Parameter, der durch die geometrische Gestaltung des Sprühkopfes wesentlich mitbestimmt wird.

Der Gestaltung der Sprühkopfgeometrie sind aus fertigungstechnischen Gründen bislang Grenzen gesetzt, so dass nicht jede zur Einstellung obiger Parameter theoretisch geeignete Geometrie auch zu wirtschaftlichen Konditionen beliebig fein herstellbar ist. Insbesondere ist bei herkömmlichen Sprühköpfen die Oberflächenqualität im Inneren der die Düsen bildenden Austrittskanäle meist unzureichend, so dass der Druckverlust in den Austrittskanälen oft zu hoch ist, und die gewünschte Zerstäubungsleistung nicht erreicht wird.

Während bei stationären Inhalationssystemen die Regulierbarkeit des Druckes und die prinzipielle Verfügbarkeit auch sehr hoher Fluiddrücke über längere Zeit zusätzliche Möglichkeiten zur Einstellung der obigen Parameter bietet, so muss bei Sprühdosen der begrenzte und mit zunehmender Gebrauchsdauer abnehmende Behälterdruck ausreichen.

Die Einsatzmöglichkeiten herkömmlicher Sprühdosen sind daher begrenzt. So eignen sich medizinische Sprays in der Regel nur zur Verabreichung einzelner kurzer Sprühstöße, einer längeren kontinuierlichen Sprühdauer sind durch den Vorrat an Sprühgut und Treibmittel Grenzen gesetzt, da die Dosierbarkeit herkömmlicher Sprays aus obigen Gründen eher grob ist, d.h. die Verringerung des austretenden Stroms an Sprühgut zugunsten einer längeren Sprühdauer für den Benutzer in der Regel nicht umsetzbar ist.

Generell scheitert die Auslegung herkömmlicher Sprühköpfe insbesondere für Sprays mit verhältnismäßig niedrigen Drücken also oft daran, dass sich eine gewünschte Tröpfchengrößenverteilung und/oder ein gewünschter Volumenstrom nicht genau genug einstellen lässt.

WO 94/07607 A1 beschreibt für die Herstellung eines für einen Hochdruck-Inhalationsapparat bestimmten Sprühkopfes, einzelne Schichten mit Laser zu bearbeiten und diese anschließend zusammen zu laminieren. Dieses Verfahren ist überaus aufwendig. In WO 2009/090084 A1 ist die Herstellung eines weiteren für einen Hochdruck-Inhalationsapparat bestimmten Sprühkopfes durch Tiefziehen eines Bleches offenbart, in welches zuvor mittels Laserbohrung Öffnungen eingebracht wurden. Mit diesem Herstellungsverfahren lässt sich nur schwer eine ausreichende Fertigungspräzision erzielen. WO 2016/075433 A1 offenbart die Fertigung eines Sprühkopfes als Spritzgussteil aus Kunststoff, wobei die Düsen mittels Laserbohrung erzeugt werden. Auch hier reicht die mögliche Fertigungspräzision kaum dazu aus, eine erwünschte Tröpfchengrößenverteilung durch exakte Auswahl der Düsengeometrie einzusteller DE 10 2006 010877 A1 offenbart eine Düse zur Zerstäubung einer flüssigen Arzneimittelformulierung. Die Düse wird aus zwei plattenförmigen Materialstücken hergestellt, wie z.B. ein Silizium-Wafer und einem zweiten Materialstück als Silizium-Wafer oder Glasscheibe. DE 20 2017 002851 U1 beschreibt einen Düsenkörper aus Glasmaterial und seine Herstellung.

Angesichts der Einschränkungen denen der Einsatz herkömmlicher Düsenkörper, insbesondere in Sprühdosen, Inhalatoren und Zerstäuberpumpen, unterliegt, liegt der vorliegenden Erfindung die Aufgabe zugrunde, die Nachteile der aus dem Stand der Technik bekannten Düsenkörper zumindest zu vermindern. Insbesondere liegt der Erfindung auch die Aufgabe zugrunde, die Auswahl an verfügbaren Geometrien für mit vertretbarem öknonomischen Aufwand herstellbare Düsenkörper im Feinstbereich zu erweitern.

Gemäß einem Aspekt der vorliegenden Erfindung wird diese Aufgabe durch einen Düsenkörper gelöst, der einen konischen oder zylindrisch-konischen, fluidisch durchströmbaren Hohlraum aufweist, welcher mindestens einen zum Hohlraummantel überwiegend tangentialen Fluideintritt und einen zum Hohlraummantel im wesentlichen axialen Fluidaustritt besitzt, wobei der Düsenkörper einstückig aus einem Glasmaterial gefertigt ist, und der geringste Durchmesser des Fluideintritts und der geringste Durchmesser des Fluidaustritts jeweils 100 Mikrometer oder kleiner ist.

Der geringste Durchmesser ist dabei jeweils der Abstand von Innenfläche zu Innenfläche des Fluidein- bzw. Fluidaustritts entlang einer geraden Linie, welche die Verbindungslinie der Flächenschwerpunkte der beiden durchströmten Flächen, die von den Rändern des Fluideintritts bzw. -austritts aufgespannt werden, orthogonal schneidet.

Als überwiegend tangential wird hier insbesondere verstanden, dass die Verbindungslinie der Flächenschwerpunkte der beiden durchströmten Flächen, die von den Rändern des Fluideintritts aufgespannt werden, an ihrem Schnittpunkt mit der Mantelfläche des Hohlraums einen größeren Tangential- als Radialanteil hat. An besagtem Schnittpunkt ist der Winkel besagter Verbindungslinie zu einer orthogonal zur Längsachse des Hohlraums liegenden Tangente also geringer, als der Winkel zu einer orthogonal auf der Längsachse stehenden, durch den Schnittpunkt gehenden Radialen. Als im wesentlichen axial wird hier insbesondere verstanden, wenn die Verbindungslinie der Flächenschwerpunkte der beiden durchströmten Flächen, die von den Rändern des Fluideintritts aufgespannt werden, von der Richtung der Längsachse des Hohlraums um weniger als 15 Grad abweicht.

Die Erfindung stellt somit insbesondere eine miniaturisierbare, aus einem Stück Glasmaterial einstückig herstellbare Hohlkegeldüse (HKD) bereit.

Durch die Verwendung von Glasmaterial, vorzugsweise Quarzglas oder Saphirglas, lässt sich eine gegenüber dem Stand der Technik erhöhte Fertigungsqualität erzielen. Insbesondere kann die Oberflächenqualität im Inneren der Austrittskanäle und an den Rändern deren Öffnungen verbessert werden, wodurch in der Massenproduktion die erzielbaren Werte für die (vom Druckverlust abhängige) Zerstäubungsleistung und den Sprühgutdurchsatz weniger stark streuen, definierte Sprüheigenschaften somit mit geringeren Abweichungen garantiert werden können.

Die nachfolgend noch näher beschriebene Fertigung in geeignetem Glas, insbesondere Quarzglas oder Saphirglas, sorgt für eine genaue Einstellbarkeit des (geringsten) Durchmessers des Fluidein- und Fluidaustritts und eine insgesamt hohe mögliche Fertigungspräzision. Insbesondere können mit diesem Material besonders gute Oberflächenqualitäten, d.h. geringe Oberflächenrauhigkeiten erzielt werden. Das Wegätzen von der Lasereinwirkung ausgesetztem Glas kann beispielsweise unter Verwendung von Flusssäure (HF) oder vorzugsweise Kalilauge (KOH) erfolgen. Das Glas kann auch geeignet dotiert sein, um die Laserbearbeitbarkeit zu verbessern. Ein solches Fertigungsverfahren ist per se unter anderem unter der Bezeichnung SLE (*Selective, Laser-Induced Etching,* selektives laserinduziertes Ätzen) bekannt und in Hermans, M. et al.: Selective, Laser-Induced Etching of Fused Silica at High Scan-Speeds Using KOH, JLMN-Journal of Laser Micro/Nanoengineering Vol. 9, No. 2, 2014 veröffentlicht. Fertigungsmaschinen, mit denen nach dem SLE-Verfahren gefertigt werden kann, sind unter der Bezeichnung *LightFab Microscanner* kommerziell erhältlich.

Vorteilhaft kann der Düsenkörper einen oder mehrere weitere zum Hohlraummantel überwiegend tangentiale Fluideintritte aufweisen, die in Umfangsrichtung und/oder axialer Richtung versetzt angeordnet sind.

Gemäß einer insbesondere für die Erzielung geringer Durchsätze bevorzugten Auführungsform ist der geringste Durchmesser des Fluideintritts 50 Mikrometer oder kleiner, vorzugsweise 20 Mikrometer oder kleiner.

Gemäß einer insbesondere für die Erzeugung feiner Tröpfchen bevorzugten Auführungsform ist der geringste Durchmesser des Fluidaustritts 50 Mikrometer oder kleiner, vorzugsweise 20 Mikrometer oder kleiner.

Gemäß einer vorteilhaften Weiterbildung weist der Düsenkörper eine Vorkammer auf, wobei der Fluideintritt von der Vorkammer zum Hohlraum fluidisch durchströmbar ist.

Gemäß einer weiteren vorteilhaften Weiterbildung weist der Düsenkörper einen Siebkörper auf, der mit dem restlichen Düsenkörper zusammen einstückig ohne den Siebkörper und den restlichen Düsenkörper verbindende Fügestelle ausgebildet ist und vorzugsweise eintrittseitig außerhalb des Hohlraums angeordnet ist.

Der Siebkörper hilft vorteilhaft, die Verstopfung des Fluideintritts oder Fluidaustritts zu vermeiden. Ferner bietet eine geeignete Wahl der Geometrie, Anzahl und Anordnungen der Sieböffnungen des Siebkörpers zusätzliche Freiheitsgrade bei der Konstruktion des Düsenkörpers, etwa um den Druckverlust oder den Durchsatz in gewünschter Weise zu beeinflussen.

Vorzugsweise sind dessen Sieböffnungen ebenfalls mittels lokaler Lasereinwirkung, insbesondere durch anschließendes Wegätzen von der lokalen Lasereinwirkung ausgesetztem Material gefertigt.

Gemäß einer besonders bevorzugten Ausführungsform ist der Fluideintritt von einer Vorkammer zum Hohlraum fluidisch durchströmbar, und der Siebkörper bildet einen Fluideintritt zur Vorkammer.

Vorzugsweise weist der Siebkörper nur Sieböffnungen auf, deren jeweiliger geringster Durchmesser (nach obiger Definition eines geringsten Durchmessers) nicht größer ist, als die Hälfte des geringsten Durchmessers des Fluideintritts, besonders bevorzugt nicht größer als ein Drittel des geringsten Durchmessers des Fluideintritts.

Vorzugsweise ist der Siebkörper in Hauptdurchströmungsrichtung der Sieböffnungen nicht dicker als das Fünffache des geringsten Durchmessers (nach obiger Definition eines geringsten Durchmessers) der Sieböffnungen (bei verschieden großen Sieböffnungen der kleinsten Sieböffnung).

Ebenfalls vorzugsweise beträgt die gesamte durchströmbare Fläche der Sieböffnungen mindestens das Hundertfache der durchströmbaren Fläche des Fluideintritts. Die durchströmbare Fläche ist dabei eine orthogonal zur Verbindungslinie der Flächenschwerpunkte der beiden durchströmten Flächen, die von den Rändern der jeweiligen Öffnung (Sieböffnung bzw. Fluideintritt) aufgespannt werden, liegende, von der Innenfläche der jeweiligen Öffnung umrandete Fläche, jeweils am Ort des jeweiligen geringsten Durchmessers.

Gemäß einem weiteren Aspekt wird eine Sprühvorrichtung bereitgestellt, welche einen Düsenkörper gemäß einem der vorangehenden Ansprüche aufweist. Ferner weist die Sprühvorrichtung eine Sprühgutbereitstellungsvorrichtung auf, welche dem Düsenkörper Sprühgut zuführt, etwa über eine Leitung bzw. ein Leitungssystem oder direkt aus einem Sprühgutbehälter, so dass dieser die Funktion der Aufbewahrung und Zuführung von Sprühgut in sich vereint. Vorteilhaft können Ventile, Schieber und sonstigen Fluidrückhalte- und/oder Fluidstromregulierungselemente vorgesehen sein um den am Düsenkörper anliegenden Druck des Sprühguts bzw. den durch den Düsenkörper austretenden Fluidstrom regulieren zu können.

Als Sprühvorrichtung wird insbesondere Sprühdose bereitgestellt, welche einen Sprühkopf besitzt, der einen erfindungsgemäßen Düsenkörper oben beschriebener Art sowie einen Sprühgutbehälter aufweist. Eine solche Sprühdose kann vorteilhaft als Pumpspray ausgeführt sein, so dass ein Nutzer, wie per se aus dem Stand der Technik bekannt, durch eine Pumpbewegung den erforderlichen Fluiddruck im Sprühgut ausüben kann, oder aber der Sprühgutbehälter enthält ein unter Druck stehendes Treibmittel, entweder in einer gemeinsamen Kammer mit dem Sprühgut oder von diesem über einen Kolben, eine Membran oder dergleichen getrennt. Auch anderweitiger mechanischer Druckaufbau insb. mittels Federsystemen ist vorteilhaft möglich.

Vorteilhafterweise kann der Sprühkopf eine Sprühkopfkulisse aus einem Kunststoff, beispielsweise Polyethylen, aufweisen, in den der Düsenkörper eingefügt ist, was dazu beiträgt, die Fertigungskosten gering zu halten. Jedoch können auch andere, beispielsweise metallische, Werkstoffe für eine Sprühkopfkulisse verwendet werden. Besonders kostengünstig kann die Sprühkopfkulisse als Spritzgussteil ausgeführt sein.

Gemäß einer besonders vorteilhaften Ausführungsform weist der Sprühkopf Adapterverbindungsmittel für einen Patientenluftwegeadapter auf, wobei der Sprühkopf mittels der Adapterverbindungsmittel werkzeugfrei, beispielsweise mittels einer Steck- oder Schraubverbindung, so mit dem Patientenluftwegeadapter verbindbar ist, dass aus dem Austrittskanal ausgetretenes Sprühgut in den Patientenluftwegeadapter eintreten kann. Ein solcher Patientenluftwegeadapter als in eine Patientennase einführbarer Nasenadapter, als Mundstück, oder als einen Patientenmund und/oder eine Patientennase abdeckender Maskenadapter ausgebildet sein.

Ebenfalls vorteilhaft kann der Sprühkopf mit einem Patientenluftwegeadapter integral geformt oder verbunden sein, der wiederum als in eine Patientennase einführbarer Nasenadapter, als einen Patientenmund und/oder eine Patientennase abdeckender Maskenadapter oder als mit einem Patientenmund umschließbarer Adapter ausgebildet ist.

Vorteilhafterweise kann der Patientenluftwegeadapter aus einem geeigneten Kunststoff wie etwa PE oder PP, beispielsweise als Spritzgussteil, ausgeführt sein.

Vorzugsweise übersteigt der Druck im Sprühbehälter der Sprühdose 30 bar nicht, bevorzugt übersteigt er 15 bar, besonders bevorzugt 10 bar nicht. Auch im Bereich von Behälterdrücken von 3 bis 10 bar erreicht die erfindungsgemäße Sprühdose noch eine wesentlich feinere Tröpfchengrößen als herkömmliche Sprühdosen mit Treibmittel.

Für Drücke von 6 bar und kleiner, insbesondere 5 bar und kleiner hat sich herausgestellt, dass für den Fachmann überraschend eine weitere Verkleinerung der in diesem Druckbereich durch Sprühen erzielbaren Tröpfchengrößen erreichen lässt, wenn der geringste Durchmesser des Fluideintritts und der geringste Durchmesser des Fluidaustritts gegenüber dem obigen Aspekt der Erfindung leicht vergrößert ist, d.h. zwischen 100 Mikrometer und 300 Mikrometer, vorzugsweise zwischen 100 Mikrometer und 250 Mikrometer beträgt. Während mit verminderten Drücken die durch die Zerstäubung erzielbaren Tröpfchendurchmesser größer werden und in höheren Druckbereichen eine Verkleinerung des geringsten Austrittsdurchmessers in der Regel eine Tröpfchenverkleinerung mit sich bringt, kann also im Durckbereich unterhalb 6 bar, insbesondere unterhalb 5 bar der Vergrößerung der möglichen Tröpfchendurchmesser gegenüber höheren Drücken entgegengewirkt werden, indem man die geringsten (insb. Austritts-)Durchmesser etwas anhebt, nämlich in den Bereich von 100 Mikrometern bis 300 Mikrometern, vorzugsweise von 100 Mikrometern bis 250 Mikrometern

Ein solcher immer noch dem Feinstbereich zuzuordnender Düsenkörper aus Glasmaterial, bei dem herkömmliche Fertigungstechnik an ihre Grenzen stößt, kann vorteilhaft wie oben beschrieben gefertigt und, abgesehen vom vergrößerten Fluidein- und Austrittsdurchmesser, wie oben beschrieben gestaltet sein. Wiederum lässt sich hier erfindungsgemäß mit Glasmaterial eine höhere Fertigungsgüte erreichen sowie eine in diesem Größenmaßstab anderweitig nicht wirtschaftlich zu erzielende Eignung für Massenproduktion.

Entsprechend stellt die vorliegende Erfindung gemäß einem weiteren Aspekt eine Sprühvorrichtung bereit, die einen Düsenkörper aufweist, der einen konischen oder zylindrisch-konischen, fluidisch durchströmbaren Hohlraum umfasst, welcher einen zum Hohlraummantel überwiegend tangentialen Fluideintritt und einen zum Hohlraummantel im wesentlichen axialen Fluidaustritt aufweist, wobei der Düsenkörper einstückig aus einem Glasmaterial gefertigt ist, und der geringste Durchmesser des Fluideintritts und der geringste Durchmesser des Fluidaustritts zwischen 100 Mikrometer und 300 Mikrometer, vorzugsweise zwischen 100 Mikrometer und 250 Mikrometer ist, und die ferner eine Sprühgutbereitstellungsvorrichtung zum Heranführen von Sprühgut an den Fluideintritt mit einem Sprühgutdruck von 6 bar oder weniger, vorzugsweise 5 bar oder weniger aufweist.

Die für den Fachmann überraschende Verkleinerung der erzielbaren Tröpfchengrößen durch Vergrößerung des geringsten Durchmessers des Fluideintritts und des Fluidaustritts auf zwischen 100 und 300 Mikrometer, wenn am Fluideintritt ein Druck des Sprühguts unter 6 bar, vorzugsweise unter 5 bar anliegt, konnte darauf zurückgeführt werden, dass sich bei feineren Ein- bzw. Austrittsdurchmessern eine Tulpenbildung des Sprühguts im Inneren des Hohlraums vollziehen kann, wenn der geringste Durchmesser des Fluideintritts und des Fluidaustritts 100 Mikrometer unterschreitet.

Für den besonders bevorzugten Anwendungsbereich der erfindungsgemäßen Sprühvorrichtungen werden diese mit zumindest einem medizinischen Wirkstoff, vorzugsweise Sole, beschickt bzw. (ggf. zusätzlich mit einem Treibmittel) befüllt. Auch wenn der erfindungsgemäße Düsenkörper hauptsächlich für Sprühdosen insbesondere für medizinische Anwendungen beschrieben wird, ist die Erfindung darauf nicht beschränkt. Vielmehr lässt sich der erfindungsgemäße Düsenkörper in einer Vielzahl von technischen Anwendungen einsetzen, insbesondere Anwendungen in welchen eine feine Zerstäubung bei niedrigen Volumenströmen und begrenzten verfügbaren Druckdifferenzen gewünscht wird, beispielsweise für die Desinfektion von Klimaanlagen oder mit Druckluft betriebene Vernebler für zur Atemluft- oder Hautoberflächenbefeuchtung in Kliniken. Auch in kosmetische Anwendungen ist eine besonders feine Zerstäubung oft wünschenswert. Hier lassen sich Sprays mit erfindungsgemäßen Düsenkörpern ebenfalls produktwertsteigernd einsetzen.

Gemäß einem weiteren Aspekt wird ein medizinischer Inhalator bereitgestellt, welcher einen Düsenkörper gemäß einem der vorangehenden Ansprüche aufweist.

Die Erfindung wird nachfolgend in beispielhafter Weise anhand der beigefügten schematischen Zeichnung näher erläutert. Die Zeichnungen sind nicht maßstabsgetreu; insbesondere entsprechen Verhältnisse der einzelnen Abmessungen zueinander aus Gründen der Anschaulichkeit teilweise nicht den Abmessungsverhältnissen in tatsächlichen technischen Umsetzungen. Es werden mehrere bevorzugte Ausführungsbeispiele beschrieben, auf welche die Erfindung jedoch nicht beschränkt ist. Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

Es zeigt
- Fig. 1a: die Querschnittsdarstellung eines erfindungsgemäßen Düsenkörpers, wobei die Schnittebene der Fig. 1b als durchbrochene Linie A-A' angedeutet ist
- Fig. 1b: eine weitere, zu Fig. 1a orthogonale Querschnittsdarstellung des Düsenkörper aus Fig. 1a, wobei die Schnittebene der Fig. 1a als durchbrochene Linie B-B' angedeutet ist,
- Fig. 1c: den Fluideintritt aus Fig. 1b als Ausschnitt zur Illustration der geometrischen Verhältnisse.
- Fig. 2a: die Querschnittsdarstellung eines weiteren erfindungsgemäßen Düsenkörpers, wobei die Schnittebene der Fig. 2b als durchbrochene Linie A-A' angedeutet ist
- Fig. 2b: eine weitere, zu Fig. 2a orthogonale Querschnittsdarstellung des Düsenkörper aus Fig. 2a, wobei die Schnittebene der Fig. 2a als durchbrochene Linie B-B' angedeutet ist, und

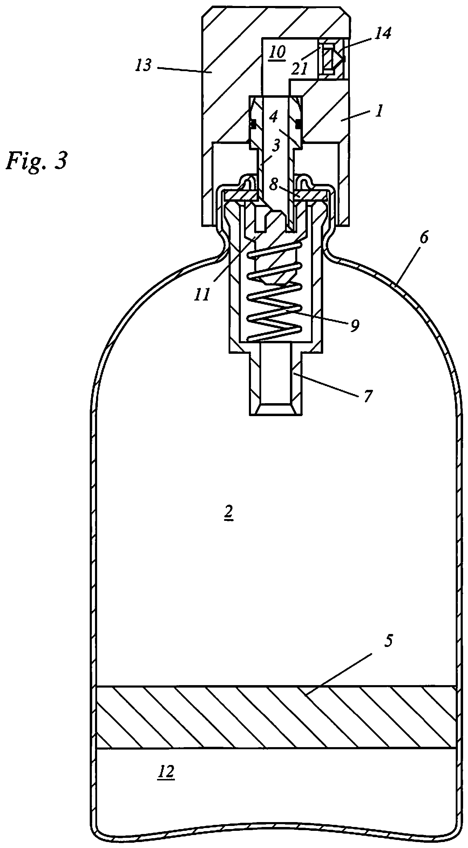

- Fig. 3: eine erfindungsgemäße Sprühdose mit seitlich austragendem Sprühkopf im Querschnitt.

Einander entsprechende Elemente sind in den Zeichnungsfiguren jeweils mit den gleichen Bezugszeichen bezeichnet.

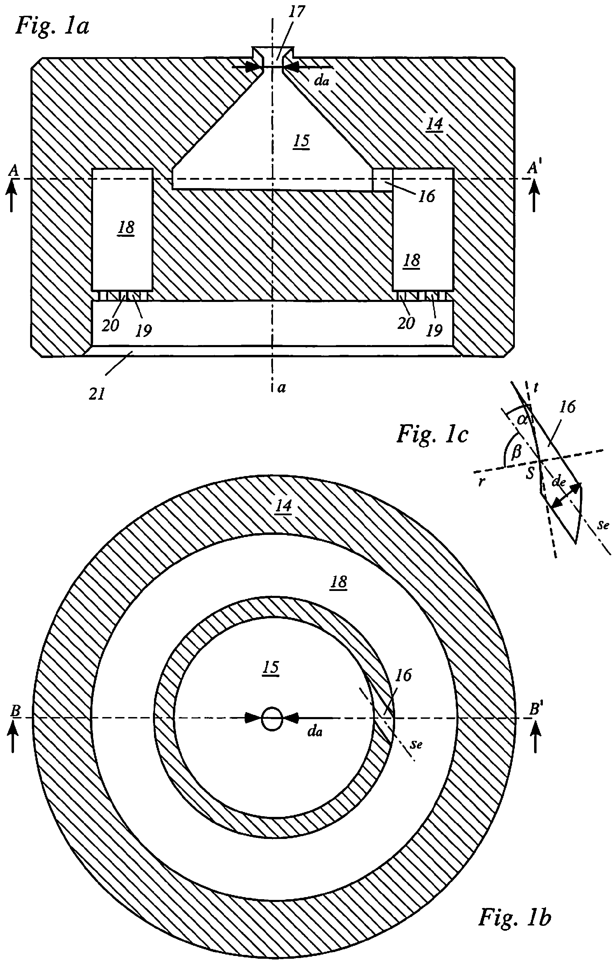

Fig. la zeigt die Querschnittsdarstellung eines erfindungsgemäßen Düsenkörpers 14, wobei die Schnittebene der Fig. 1b als durchbrochene Linie A-A' angedeutet ist. Die Schnittebene der Fig. 1a ist in Fig. 1a als durchbrochene Linie B-B' angedeutet. Die Schnittebenen der Figuren 1a und 1b stehen also senkrecht aufeinander.

Mittels selektiver Laserbelichtung und anschließendem Ausätzen der belichteten Bereiche (selektives laserinduziertes Ätzen) ist in dem Quarzglas-Düsenkörper 14 mit zylindrischer Grundform ein zylindrisch-konischer Hohlraum 15 mit weitgehend tangentialem Fluideintritt 16 und axialem Fluidaustritt 17 gefertigt.

Der Fluideintritt 16 ist in Fig. 1c vergrößert dargestellt. Die Symmetrieachse se des Fluideintritts 16 verbindet die Flächenschwerpunkte der von den Rändern des Fluideintritts 16 aufgespannten durchströmbaren Flächen. Der Winkel α, den die Symmetrieachse se mit der Tangente t an die Mantelfläche des Hohlraums 15 bildet, ist kleiner als der Winkel β, den die Symmetrieachse se mit der am Schnittpunkt S mit der Tangente t orthogonal zur Mantelfläche stehenden radialen Gerade r bildet.

Der jeweils geringste Durchmesser des Fluideintritts 16 de und Fluidaustritts 17 dₐ beträgt jeweils unter 100 µm. Mit dem genannten Herstellungsverfahren des selektiven laserinduzierten Ätzens lassen sich auch Durchmesser von nur wenigen Mikrometern erreichen.

Die Symmetrieachse des kegeligen Abschnitts des Hohlraums 15 ist auch Symmetrieachse des Fluidaustritts 17. Der Fluidaustritt 17 endet im dargestellten Beispiel mit einer konischen Erweiterung und einer scharfen Abrisskante, um die Fluidzerstäubung zu begünstigen.

Zugeführt wird zu zerstäubende Flüssigkeit dem Fluideintritt 16 über den Zulauf 21 und die ringförmige Vorkammer 18. Zwischen dem Zulauf 21 und der Vorkammer 18 durchströmt die zu zerstäubende Flüssigkeit den (nicht zwingend vorgesehenen) Siebkörper 19. Dessen Öffnungen 20 besitzen einen jeweils geringsten Durchmesser von einem Drittel des geringsten Durchmessers de des Fluideintritts 16. Der Siebkörper 19 bewahrt den Fluideintritt 16 und den Fluidaustritt 17 vor Verstopfen.

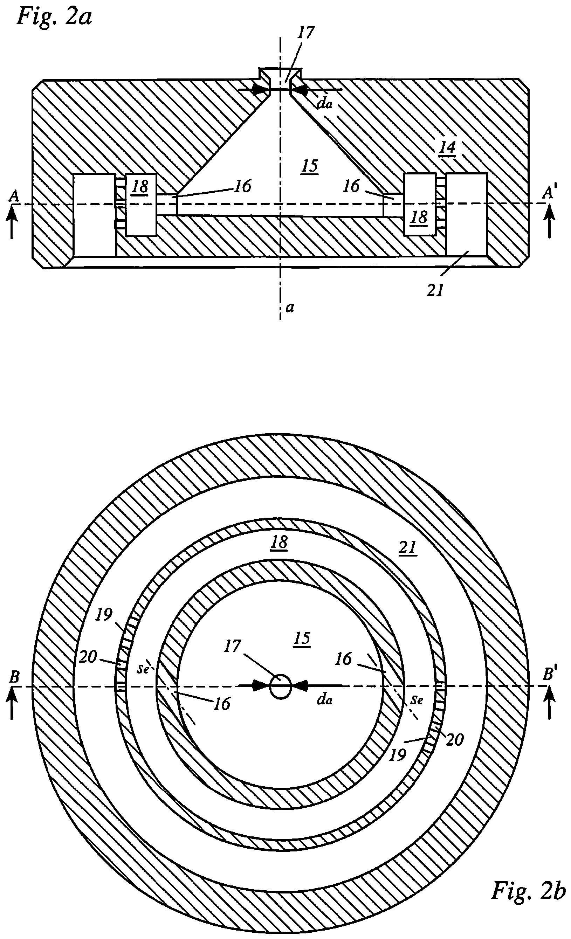

Über den Umfang können auch mehrere Fluideintritte 16 verteilt sein, wie in Fig. 2a dargestellt. In diesem Ausführungsbeispiel ist der Siebkörper 19 radial zur Vorkammer 18 angeordnet und nicht axial, wie in Fig. 1a. Anders als in Fig. 2b dargestellt, kann statt der umlaufenden Vorkammer 18 auch jeweils eine separate Vorkammer für jeden Fluideintritt 16 vorgesehen sein.

Die Sprühdose in Fig. 2 weist einen Sprühkopf 1 auf, in dessen Sprühkopfkulisse 13 der Düsenkörper 14 so eingesetzt ist, dass der Sprühgutaustrag zur Seite erfolgt. Eine derarartige Anordnung ist für vielerlei technische (beispielsweise zur Desinfektion) oder kosmetische (z.B. zur Hautbefeuchtung oder zum Auftrag von Duftstoffen) Anwendungen vorteilhaft.

Der wiederverwendbare Sprühkopf 1 ist auf den Kopf 4 des Austrittsrohrs 3 des Sprühbehälters 2 aufgesetzt und von diesem werkstofffrei abziehbar, um den Austausch des Sprühbehälters 2 zu ermöglichen. Alternativ ist auch eine Ausführung als Einwegprodukt möglich.

Der Sprühbehälter 2 ist als Zweikammerbehälter ausgeführt, in welchem ein verschieblicher Kolben 5 das im unteren Bereich 12 des Sprühbehälters 2 vorhandene komprimierte Treibmittel, beispielsweise Stickstoff, vom Sprühgut, beispielsweise Sole, trennt. Alternativ lässt sich die Erfindung auch mit einem Einkammerbehälter als Sprühbehälter 2 ausführen, bei welchem Treibmittel und Sprühgut vermischt sind. Als Treibmittel eignen sich die aus herkömmlichen Sprays bekannten Treibmittel. Die Behälterwand 6 des Sprühbehälters 2 ist aus herkömmlichem Behältermaterial wie beispielsweise Aluminium oder Weißblech gefertigt.

Das Ventil des Sprühbehälters 2 ähnelt den Ventilen herkömmlicher Sprühdosen und weist ein Ventilgehäuse 7 auf, welches über den aus einem Elasten wie Kautschuk- oder Silikongummi bestehenden Dichtring 8 abgedichtet ist. Die ins Ventilgehäuse eingesetzte Feder 9 drückt die Verschlusskapsel 11 gegen den Dichtring 8. Durch Zusammendrücken von Sprühkopf 1 und und Sprühbehälter 2 relativ zu einander schiebt das unten angeschrägte Austrittsrohr 3 die Verschlusskapsel nach unten, so dass Sprühgut durch Ventilgehäuse 7 und Austrittsrohr 3 in den Zuführungskanal 10 des Sprühkopfs 1 eintreten kann.

Der Sprühkopfkulisse 13 besteht aus thermoplastischem oder duroplastischen Kunststoff, z.B. Polyethylen, Polypropylen oder Polycarbonat. Eingesetzt in die Sprühkopfkulisse 13 ist der Düsenkörper 14, welcher in Fig. 1a und 1b vergrößert dargestellt ist.

## Patentansprüche

1. Düsenkörper (14) aufweisend
einen konischen oder zylindrisch-konischen, fluidisch durchströmbaren Hohlraum (15), welcher einen zum Hohlraummantel überwiegend tangentialen Fluideintritt (16) und einen zum Hohlraummantel im wesentlichen axialen Fluidaustritt (17) aufweist,
wobei der Düsenkörper (14) einstückig aus einem Glasmaterial gefertigt ist, und
der geringste Durchmesser des Fluideintritts (16) und der geringste Durchmesser des Fluidaustritts (17) jeweils 100 Mikrometer oder kleiner ist.

2. Düsenkörper gemäß einem der vorangehenden Ansprüche, wobei der geringste Durchmesser des Fluideintritts 50 Mikrometer oder kleiner ist.

3. Düsenkörper gemäß einem der vorangehenden Ansprüche, wobei der geringste Durchmesser des Fluideintritts 20 Mikrometer oder kleiner ist.

4. Düsenkörper gemäß einem der vorangehenden Ansprüche, wobei der geringste Durchmesser des Fluidaustritts 50 Mikrometer oder kleiner ist.

5. Düsenkörper gemäß einem der vorangehenden Ansprüche, wobei der geringste Durchmesser des Fluidaustritts 20 Mikrometer oder kleiner ist.

6. Düsenkörper gemäß einem der vorangehenden Ansprüche, wobei der Fluideintritt und der Fluidaustritt mittels lokaler Lasereinwirkung gefertigt sind.

7. Düsenkörper gemäß Anspruch 6, wobei der Fluideintritt und der Fluidaustritt durch lokale Lasereinwirkung und anschließendes Wegätzen von der lokalen Lasereinwirkung ausgesetztem Material gefertigt sind.

8. Düsenkörper (14) gemäß einem der vorangehenden Ansprüche, wobei das Glasmaterial Quarzglas ist.

9. Sprühvorrichtung, aufweisend einen Düsenkörper (14) gemäß einem der vorangehenden Ansprüche.

10. Sprühvorrichtung, aufweisend
einen Düsenkörper (14), der einen konischen oder zylindrisch-konischen, fluidisch durchströmbaren Hohlraum (15) aufweist, welcher einen zum Hohlraummantel überwiegend tangentialen Fluideintritt (16) und einen zum Hohlraummantel im wesentlichen axialen Fluidaustritt (17) aufweist, wobei der Düsenkörper (14) einstückig aus einem Glasmaterial gefertigt ist, und der geringste Durchmesser des Fluideintritts und der geringste Durchmesser des Fluidaustritts (17) zwischen 100 Mikrometer und 300 Mikrometer, vorzugsweise zwischen 100 Mikrometer und 250 Mikrometer ist,
eine Sprühgutbereitstellungsvorrichtung zum Heranführen von Sprühgut an den Fluideintritt (16) mit einem Sprühgutdruck von 6 bar oder weniger, vorzugsweise 5 bar oder weniger.

11. Sprühvorrichtung gemäß einem der Ansprüche 9 oder 10, welche als Sprühdose ausgeführt ist, aufweisend einen Sprühgutbehälter (2) und einen Sprühkopf (1), welcher den Düsenkörper (14) aufweist.

12. Sprühvorrichtung gemäß Anspruch 11, welches als Pumpspray ausgeführt ist.

13. Sprühvorrichtung gemäß Anspruch 11, wobei der Sprühgutbehälter (2) ein unter Druck stehendes Treibmittel enthält.

14. Sprühvorrichtung gemäß einem der Ansprüche 11 oder 12, welche als medizinischer Inhalator ausgeführt ist.

15. Verfahren zum Herstellen eines einstückigen Düsenkörpers (14), der einen konischen oder zylindrisch-konischen, fluidisch durchströmbaren Hohlraum (15) aufweist, aus einem Glasmaterial,
wobei ein zum Hohlraummantel überwiegend tangentialer Fluideintritt (16) in den Hohlraum (15) und ein zum Hohlraummantel im wesentlichen axialer Fluidaustritt (17) aus dem Hohlraum durch lokale Lasereinwirkung und anschließendes Wegätzen von der lokalen Lasereinwirkung ausgesetztem Material gefertigt werden, dass der geringste Durchmesser des Fluideintritts (16) und der geringste Durchmesser des Fluidaustritts (17) jeweils 100 Mikrometer oder kleiner ist.

## Claims

1. A nozzle body (14) comprising
a conical or cylindrical-conical cavity (15) through which fluid can flow and which comprises a fluid inlet (16) predominantly tangential relative to the cavity jacket and a fluid outlet (17) substantially axial relative to the cavity jacket,
the nozzle body (14) being formed in one piece from a glass material, and
the smallest diameter of the fluid inlet (16) and the smallest diameter of the fluid outlet (17) each being 100 micrometers or less.

2. The nozzle body according to one of the preceding claims, wherein the smallest diameter of the fluid inlet is 50 micrometers or less.

3. The nozzle body according to one of the preceding claims, wherein the smallest diameter of the fluid inlet is 20 micrometers or less.

4. The nozzle body according to any one of the preceding claims, wherein the minimum diameter of the fluid outlet is 50 micrometers or less.

5. The nozzle body according to any one of the preceding claims, wherein the minimum diameter of the fluid outlet is 20 micrometers or less.

6. The nozzle body according to any one of the preceding claims, wherein the fluid inlet and fluid outlet are formed by means of local laser exposure.

7. The nozzle body according to claim 6, wherein the fluid inlet and the fluid outlet are formed by local laser exposure and subsequent etching away of material exposed to the local laser exposure.

8. The nozzle body (14) according to any of the preceding claims, wherein the glass material is quartz glass.

9. A spraying device comprising a nozzle body (14) according to any one of the preceding claims.

10. A spraying device comprising
a nozzle body (14) which comprises a conical or cylindrical-conical cavity (15) through which fluid can flow and which comprises a fluid inlet (16) which is predominantly tangential relative to the cavity jacket and a fluid outlet (17) which is substantially axial relative to the cavity jacket, wherein the nozzle body (14) is formed in one piece from a glass material, and the smallest diameter of the fluid inlet and the smallest diameter of the fluid outlet (17) is between 100 micrometers and 300 micrometers, preferably between 100 micrometers and 250 micrometers,
a spray material supply device for supplying spray material to the fluid inlet (16) with a spray material pressure of 6 bar or less, preferably 5 bar or less.

11. The spraying device according to any one of claims 9 and 10, said spraying device being configured as a spray can, comprising a spray material container (2) and a spray head (1) comprising the nozzle body (14).

12. The spraying device according to claim 11, said spraying device being configured as a pump spray.

13. The spraying device according to claim 11, wherein the spray material container contains a pressurized propellant.

14. The spraying device according to any one of claims 11 and 12, said spraying device being configured as a medical inhaler.

15. A method for manufacturing, from a glass material, a nozzle body (14) in one piece, which has a conical or cylindrical-conical cavity (15) through which fluid can flow,
wherein a, relative to the cavity jacket, predominantly tangential fluid inlet (16) into the cavity and a, relative to the cavity jacket, substantially axial fluid outlet (17) from the cavity are produced by local laser exposure and subsequent etching away of material exposed to the local laser exposure, such that the smallest diameter of the fluid inlet (16) and the smallest diameter of the fluid outlet (17) each are 100 micrometers or less.

## Revendications

1. Corps d'ajutage (14) comprenant
une cavité (15) conique ou cylindro-conique, pouvant être traversée par un fluide, qui comprend une entrée de fluide (16), globalement tangentielle par rapport à l'enveloppe extérieure de la cavité, et une sortie de fluide (17) globalement axiale par rapport à l'enveloppe extérieure de la cavité,
dans lequel le corps d'ajutage (14) est réalisé d'une seule pièce à partir d'un matériau de type verre et
le diamètre le plus petit de l'entrée de fluide (16) et le diamètre le plus petit de la sortie de fluide (17) sont respectivement de 100 micromètres ou moins.

2. Corps d'ajutage selon l'une des revendications précédentes, dans lequel le diamètre le plus petit de l'entrée de fluide est de 50 micromètres ou moins.

3. Corps d'ajutage selon l'une des revendications précédentes, dans lequel le diamètre le plus petit de l'entrée de fluide est de 20 micromètres ou moins.

4. Corps d'ajutage selon l'une des revendications précédentes, dans lequel le diamètre le plus petit de la sortie de fluide est de 50 micromètres ou moins.

5. Corps d'ajutage selon l'une des revendications précédentes, dans lequel le diamètre le plus petit de la sortie de fluide est de 20 micromètres ou moins.

6. Corps d'ajutage selon l'une des revendications précédentes, dans lequel l'entrée de fluide et la sortie de fluide sont réalisées à l'aide de l'action locale d'un laser.

7. Corps d'ajutage selon la revendication 6, dans lequel l'entrée de fluide et la sortie de fluide sont réalisées à l'aide de l'action locale d'un laser puis d'un enlèvement par gravure du matériau exposé par l'action locale du laser.

8. Corps d'ajutage (14) selon l'une des revendications précédentes, dans lequel le matériau de type verre est du verre de quartz.

9. Dispositif de pulvérisation comprenant un corps d'ajutage (14) selon l'une des revendications précédentes.

10. Dispositif de pulvérisation comprenant
un corps d'ajutage (14), qui comprend une cavité (15) conique ou cylindro-conique, pouvant être traversée par un fluide, qui comprend une entrée de fluide (16), globalement tangentielle par rapport à l'enveloppe extérieure de la cavité, et une sortie de fluide (17) globalement axiale par rapport à l'enveloppe extérieure de la cavité, dans lequel le corps d'ajutage (14) est réalisé d'une seule pièce à partir d'un matériau de type verre et le diamètre le plus petit de l'entrée de fluide et le diamètre le plus petit de la sortie de fluide (17) sont entre 100 micromètres et 300 micromètres, de préférence entre 100 micromètres et 250 micromètres,
un dispositif de mise à disposition de produit à pulvériser pour amener le produit à pulvériser vers l'entrée de fluide (16) avec une pression de produit à pulvériser de 6 bar ou moins, de préférence de 5 bar ou moins.

11. Dispositif de pulvérisation selon l'une des revendications 9 ou 10, qui est conçu comme une bombe aérosol, comprenant un récipient de produit à pulvériser (2) et une tête de pulvérisation (1), qui comprend le corps d'ajutage (14).

12. Dispositif de pulvérisation selon la revendication 11, qui est conçu comme un vaporisateur.

13. Dispositif de pulvérisation selon la revendication 11, dans lequel le récipient de produit à pulvériser (2) contient un gaz propulseur sous pression.

14. Dispositif de pulvérisation selon l'une des revendications 11 ou 12, qui est conçu comme un inhalateur médical.

15. Procédé de fabrication d'un corps d'ajutage (14) d'une seule pièce, qui comprend une cavité (15) conique ou cylindro-conique, pouvant être traversée par un fluide, à partir d'un matériau de type verre,
dans lequel une entrée de fluide (16), globalement tangentielle par rapport à l'enveloppe extérieure de la cavité est réalisée dans la cavité (15) et une sortie de fluide (17) globalement axiale par rapport à l'enveloppe extérieure de la cavité, est réalisée à partir de la cavité, par l'action locale d'un laser puis l'enlèvement par gravure du matériau exposé par l'action locale du laser, de façon à ce que le diamètre le plus petit de l'entrée de fluide (16) et le diamètre le plus petit de la sortie de fluide (17) soient respectivement de 100 micromètres ou moins.
